(19) Europäisches Patentamt
European Patent Office
Office européen des brevets

(11) Veröffentlichungsnummer : **0 202 523 B1**

(12) # EUROPÄISCHE PATENTSCHRIFT

(45) Veröffentlichungstag der Patentschrift : **30.01.91 Patentblatt 91/05**

(51) Int. Cl.$^5$ : **C07C 271/10,** C07C 269/00

(21) Anmeldenummer : **86106091.1**

(22) Anmeldetag : **03.05.86**

(54) **Verfahren zur Herstellung von 1,3-Di(alkoxicarbonylamino)propanen.**

(30) Priorität : **11.05.85 DE 3517110**

(43) Veröffentlichungstag der Anmeldung : **26.11.86 Patentblatt 86/48**

(45) Bekanntmachung des Hinweises auf die Patenterteilung : **30.01.91 Patentblatt 91/05**

(84) Benannte Vertragsstaaten : **BE DE FR GB IT NL**

(56) Entgegenhaltungen : **DE-B- 1 158 492**

(73) Patentinhaber : **BASF Aktiengesellschaft
Carl-Bosch-Strasse 38
D-6700 Ludwigshafen (DE)**

(72) Erfinder : **Merger, Franz, Dr.
Max-Slevogt-Strasse 25
D-6710 Frankenthal (DE)**
Erfinder : **Liebe, Jörg, Dr.
Hans-Purrmann-Strasse 10
D-6710 Frankenthal (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft ein Verfahren zur Herstellung von unsubstituierten oder substituierten 1,3-Di(alkoxicarbonylamino)propanen durch Umsetzung von $\alpha$,ß-ungesättigten Aldehyden mit N-unsubstituierten Carbaminsäureestern in Gegenwart saurer Katalysatoren zu 1,1,3-Tri(alkoxicarbonylamino)propanen und deren hydrogenolytische Spaltung bei erhöhter Temperatur und Druck in Gegenwart von Hydrierkatalysatoren.

Substituierte 1,3-Di(alkoxicarbonylamino)propane (Propan-1,3-diurethane) lassen sich zu Propan-1,3-diaminen verarbeiten und sind andererseits wichtige Ausgangsmaterialien zur Herstellung von Wirkstoffen oder deren Vorprodukte, wie z.B. von 2-Oxohexahydropyrimidinen. Außerdem lassen sich 1,3-Di(alkoxicarbonylamino)propane thermisch zu 1,3-Diisocyanaten spalten, die als Monomere mit hoher Isocyanatzahl für die Herstellung von Polyurethanen Verwendung finden können.

Die Umsetzung von N-unsubstituierten Carbaminsäureestern mit $\alpha$,ß-ungesättigten Aldehyden unter Bildung von 1,1,3-Tri(alkoxicarbonylamino)propanen ist im Chem. Rev. 65, 583 (1965) beschrieben. Die Ausbeuten bei den beschriebenen Reaktionen liegen bei 20 bis 40%, als Katalysator wurde Salzsäure eingesetzt. In zwei weiteren Veröffentlichungen, Glasnik Hemijskog Drustva, Beograd, 34, 387 bis 394 (1969), ibid 41, 219 bis 224 (1976) wird die Synthese einiger spezieller 1,1,3-Tri(alkoxicarbonylamino)propane beschrieben. Auch dort werden Salzsäure sowie Chlorwasserstoff und Bortrifluorid-Etherat, also sehr korrosive Reagenzien, als Katalysatoren eingesetzt. Bei der Aufarbeitung muß mit Wasser gewaschen werden, um die Säure zu entfernen. Die Ausbeuten an Triurethanen, von einer speziellen Ausnahme abgesehen (80%), liegen im Bereich von 20 bis 50%.

Substituierte 1,3-Di(alkoxicarbonylamino)propane lassen sich durch Umsetzung von 1,3-Diaminopropanen mit Chlorameisensäureestern herstellen, vgl. Houben-Weyl, 4. Auflage, Band VIII, Seite 138. Dieses Verfahren besitzt den Nachteil, daß schlecht zugängliche Edukte, wie die 1,3-Diaminopropan-Verbindungen benötigt werden. Zusätzlich fallen als Nebenprodukt bei der Reaktion größere Mengen Chlorwasserstoff an.

In der DE-A-11 58 492 wird die Umsetzung von 1,1-Alkylidendiurethanen mit kationisch polymerisierbaren Mono- und/oder Polyolefinen beschrieben. Bei diesem Verfahren entstehen stets Gemische aus dem gewünschten 1,3-Diurethan und 1,5- sowie höheren Diurethanen.

Der Erfindung liegt die Aufgabe zugrunde, ein Verfahren der eingangs genannten Art bereitzustellen, das die Herstellung von 1,3-Di(alkoxicarbonylamino)propanen bei großer Produktreinheit und ohne Verwendung chlorhaltiger bzw. korrosiver Ausgangsverbindungen erlaubt.

Diese Aufgabe wird gelöst mit einem Verfahren zur Herstellung von 1,3-Di(alkoxicarborylamino)propanen der allgemeinen Formel I

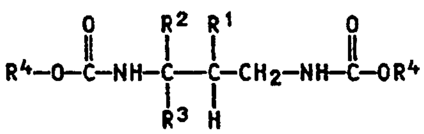

(I),

worin $R^1$, $R^2$ und $R^3$ gleich oder verschieden sein können und jeweils für ein Wasserstoffatom, einen aliphatischen, cycloaliphatischen, araliphatischen oder aromatischen Rest stehen und $R^4$ einen aliphatischen, cycloaliphatischen oder araliphatischen Rest bedeutet,
durch Umsetzung eines $\alpha,\beta$-ungesättigten Aldehyds mit einem Carbaminsäureester in Gegenwart eines sauren Katalysators, das dadurch gekennzeichnet ist, daß man in einer ersten Stufe als $\alpha,\beta$-ungesättigten Aldehyd eine Verbindung der allgemeinen Formel II

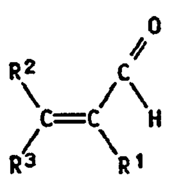

(II),

worin $R^1$, $R^2$ und $R^3$ die vorstehenden Bedeutungen besitzen, mit einem Carbaminsäureester der allgemeinen Formel III

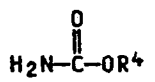 (III),

worin $R^4$ die vorstehende Bedeutung besitzt, bei einer Temperatur von 0 bis 150°C in Gegenwart eines sauren Ionenaustauscherharzes als Katalysator zu einem 1,1,3-Tri(alkoxicarbonylamino)propan der allgemeinen Formel IV

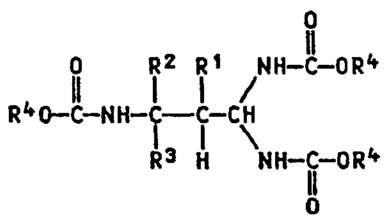 (IV),

worin $R^1$, $R^2$, $R^3$ und $R^4$ die vorstehenden Bedeutungen besitzen, umsetzt, und in einer zweiten Stufe die Verbindung der allgemeinen Formel (IV) in Gegenwart eines Hydrierkatalysators und Wasserstoff bei einem Druck von 1 bis 300 bar auf eine Temperatur von 100 bis 300°C erhitzt.

Die Umsetzung kann für den Fall der Verwendung von Crotonaldehyd und Ethylcarbamat durch die folgenden Formeln wiedergegeben werden :

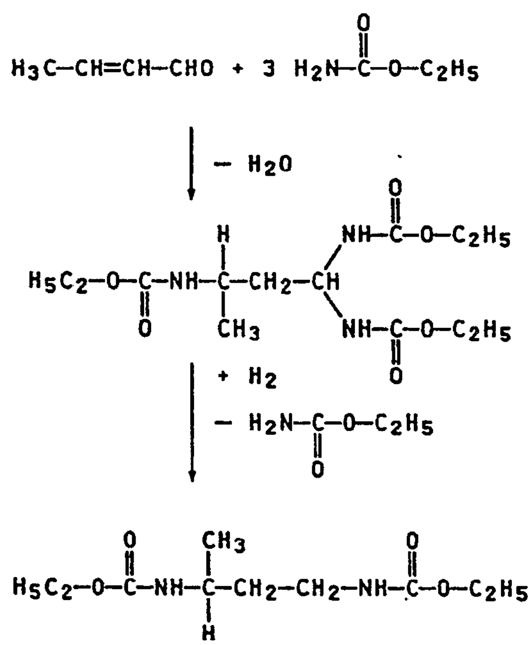

Man erhält auf diesem Weg auf überraschend einfache und wirtschaftliche Weise substituierte 1,3-Di(alkoxicarbonylamino)propane aus gut zugänglichen Edukten ohne Verwendung von chlorhaltigen bzw. korrosiven Reagenzien und damit verbundenem Anfall von chlorhaltigen Nebenprodukten. Ein weiterer Vorteil dieses Verfahrens liegt in der großen Reinheit der erhalteren Produkte.

Bevorzugt verwendet man Ausgangsstoffe der allgemeinen Formeln II und, III, worin die Reste $R^1$, $R^2$, $R^3$ gleich oder verschieden sein können und einen Alkylrest mit 1 bis 30, insbesondere 1 bis 12 Kohlenstoffatomen, einen Cycloalkylrest, einen Aralkylrest mit 7 bis 12 Kohlenstoffatomen, einen Phenylrest oder ein Wasserstoffatom bezeichnen. Die vorgenannten Reste können zusätzlich durch unter den Reaktionsbedingungen inerte Gruppen, insbesondere Alkylgruppen oder Alkoxigruppen, mit jeweils 1 bis 4 Kohlenstoffatomen substituiert sein. $R^4$ in der allgemeinen Formel III steht bevorzugt für einen Alkylrest mit 1 bis 12 Kohlenstoffatomen, einen Cycloalkylrest oder einen Aralkylrest mit 7 bis 12 Kohlenstoffatomen. Diese Reste können zusätzlich durch unter den Reaktionsbedingungen inerte Gruppen, insbesondere Alkylgruppen oder Alkoxigruppen, mit jeweils 1 bis 4 Kohlerstoffatomen, substituiert sein. Die entsprechenden Zwischenprodukte der allgemeinen Formel IV und die Endprodukte der allgemeinen Formel I sind besonders bevorzugte Verfahrensprodukte.

Geeignete Ausgangsstoffe der allgemeinen Formel II sind beispielsweise : Acrolein, Methacrolein, Ethylacrolein und höhere Homologe, Crotonaldehyd, 2-Methylpentenal, Tiglinaldehyd, Zimtaldehyd und 2-Benzylacrolein.

Geeignete Ausgangsstoffe der allgemeinen Formel III sind beispielsweise : Methyl-, Ethyl-, Propyl-, Isopropyl-, Butyl-, Isobutyl-, sek.Butyl-, Pentyl-, 3-Methylbutyl-, Hexyl-, 2-Ethylhexyl-, Heptyl-, Octyl-, Nonyl-, Decyl-, Undecyl-, Dodecyl-, Cyclohexyl-, Cyclopentyl-, Benzyl- und Methoxiethylcarbamat.

Die N-unsubstituierten aliphatischen Carbamate der Formel III können ohne Verwendung von Phosgen aus Harnstoff und dem entsprechenden Alkohol hergestellt werden. Bevorzugt sind Methyl-, Ethyl-, Propyl-, n-Butyl- und i-Butylcarbamat.

Die Umsetzung der 1. Stufe, die Herstellung des Zwischenproduktes der allgemeinen Formel IV, wird bei einer Temperatur von 0 bis 150°C, vorzugsweise zwischen 20 und 120°C, insbesondere 50 bis 90°C, drucklos oder unter Druck, diskontinuierlich oder kontinuierlich durchgeführt. Falls man unter Druck arbeitet, sind Werte von 1 bis 10 bar besonders günstig. Zweckmäßig arbeitet man in einem unter den Reaktionsbedingungen inerten Lösungsmittel, der Einsatz eines Lösungsmittels ist aber nicht zwingend. Als Lösungsmittel eignen sich : aromatische Kohlenwasserstoffe wie z.B. Benzol, Toluol, Ethylbenzol, o-, m-, p-Xylol, Isopropylbenzol ; Halogenkohlenwasserstoffe, insbesondere Chlorkohlenwasserstoffe wie z.B. Methylenchlorid, Chloroform, Tetrachlorkohlenstoff, Tetrachlorethylen, 1,1,2,2- oder 1,1,1,2-Tetrachlorethan, Amylchlorid, Dichlorpropan, Dichlorbutan, 1,1,1-oder 1,1,2-Trichlorethan, Trichlorethylen, Pentachlorethan, 1,2-Dichlorethan, 1,1-Dichlorethan, n-Propylchlorid, 1,2-cis-Dichlorethylen, Chlorbenzol, o-, m-, p-Dichlorbenzol, o-, m-, p-Chlortoluol ; Ether, z.B. Diethylether, Ethylpropylether, n-Butylethylether, Di-n-butylether, Diisobutylether, Diisoamylether, Diisopropylether, Cyclohexylmethylether, Ethylenglykoldimethylether, Tetrahydrofuran, Dioxan ; aliphatische oder cycloaliphatische Kohlenwasserstoffe, z.B. Heptan, Nonan, Benzinfraktionen innerhalb eines Siedepunktintervalls von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan ; und entsprechende Gemische. Das Lösungsmittel wird in einer Menge von 50 bis 10000 Gew.-%, vorzugsweise von 100 bis 1000 Gew.-%, bezogen auf Ausgangsstoff II, eingesetzt. Zweckmäßig ist es, das Lösungsmittel so zu wählen, daß die Siedetemperatur des Lösungsmittels mit der Reaktionstemperatur übereinstimmt.

Die Umsetzung der ersten Stufe wird in Gegenwart eines sauren Ionenaustauscherharzes als Katalysator durchgeführt. Die Menge an Katalysator kann zwischen 0,001 und 0,2, insbesondere zwischen 0,01 und 0,15 Äquivalenten, bezogen auf 1 mol Ausgangsstoff II, liegen.

Die sauren Ionenaustauscherharze gestatten eine problemlose Aufarbeitung, da sie nach Beendigung der Reaktion nur abfiltriert zu werden brauchen und ohne Ausbeuteverlust wieder eingesetzt werden können.

Bevorzugte saure Ionenaustauscherharze sind Duolite ® C 265, Lewasorb ® AC ID, Lewatit ® SPC 118.

Zur Durchführung der Reaktion der ersten Stufe wird ein Gemisch aus Lösungsmittel, saurem Ionenaustauscherharz und Verbindung der allgemeinen Formel III auf die Reaktionstemperatur gebracht. Innerhalb von 0,1 bis 5 Stunden tropft man bei dieser Temperatur die Verbindung der allgemeinen Formel II, eventuell gelöst in demselben oder einem anderen Lösungsmittel, zu und rührt ohne Abkühlung noch 0,5 bis 25 Stunden nach. Das bei der Reaktion freiwerdende Wasser stört die Reaktion nicht, kann aber auch durch azeotrope Destillation entfernt werden. Die Aufarbeitung geschieht auf üblichem Wege, z.B. durch Destillation und/oder Kristallisation.

Die Verbindung der allgemeinen Formel IV kann auch ohne Isolierung für die 2. Stufe, die hydrierende Spaltung, eingesetzt werden.

Zweckmäßigerweise arbeitet man zur Vermeidung von Polymerisationsverlusten in Gegenwart von kleinen Mengen üblicher Polymerisationsinhibitoren, wie z.B. Hydrochinon, Hydrochinonmonomethylether oder Phenothiazin.

Die Reaktion der 2. Stufe, die hydrierende Spaltung, erfolgt durch Erhitzen der Verbindung der allgemeinen Formel IV in einem bei der Reaktion inerten Lösungsmittel auf eine Temperatur von 100 bis 300°C, vorzugsweise 130 bis 200°C, besonders bevorzugt 150 bis 180°C, in Gegenwart von Wasserstoff und üblichen Hydrierkatalysatoren. Die Reaktion wird unter einem Druck von 1 bis 300 bar, bevorzugt bei 10 bis 200 bar, besonders bevorzugt bei 30 bis 100 bar, durchgeführt und ist nach 1 bis 50 Stunden beendet. Die Aufarbeitung erfolgt in üblicher Weise, z.B.durch Abfiltrieren des Katalysators, Destillation und oder Kristallisation des Rückstands. Die bei der Reaktion freiwerdende Ausgangsverbindung der allgemeinen Formel III kann wiederverwendet werden.

Als Reaktoren sind z.B. einfach Stahlautoklaven geeignet. Die Reaktion kann diskontinuierlich, aber auch kontinuierlich durchgeführt werden.

Alle üblichen Hydrierkatalysatoren sind für die Reaktion der Stufe 2 geeignet, wie z.B. Raney-Nickel, Raney-Cobalt, aber auch Edelmetallkatalysatoren wie Palladium auf Kohle, Platin auf Kohle. Der Katalysator wird bei diskontinuierlicher Fahrweise in Mengen von 5 bis 50 Gew.-%, bevorzugt 10 bis 30 Gew.-%, bezogen auf die verwendete Menge an Verbindung IV, eingesetzt.

Als besonders geeignete Zwischenprodukte der allgemeinen Formel IV kommen solche in Betracht, die aus den als bevorzugt bezeichneten Ausgangsverbindungen der allgemeinen Formeln II und III hergestellt werden.

Als Lösungsmittel für die Reaktion der Stufe 2 können alle unter den Reaktionsbedingungen inerten Lösungsmittel verwendet werden ; bevorzugt sind Alkanole und Cycloalkanole wie Ethanol, Methanol, n-Butanol, Isobutanol, n-Propanol, Isopropanol, Amylalkohol, Cyclohexanol, 2-Methyl-4-pentanol, Ethylenglykolmonoethylether, 2-Ethylhexanol, Methylglykol, n-Hexanol, Isohexylalkohol, Isoheptylalkohol, n-Heptanol, Ethylbutanol, Nonylalkohol, Methylcyclohexanol, insbesondere solche mit 1 bis 8 Kohlenstoffatomen.

Die Gesamtreaktion II + III → IV → I kann vorzugsweise unter Isolierung des Zwischenproduktes der allgemeinen Formel IV durchgeführt werden, eine solche Isolierung ist aber nicht immer erforderlich.

Die erfindungsgemäß herstellbaren 1,3-Di(alkoxicarbonylamino)propane der allgemeinen Formel I sind z.B. wertvolle Ausgangsstoffe für die Herstellung von substituierten Propan-1,3-diaminen, die sonst teilweise nur sehr schlecht zugänglich sind. Die Diamine lassen sich nach bekannten Methoden, z.B. durch alkalische Verseifung, aus den 1,3-Diurethanen einfach herstellen. Die 1,3-Diurethane bieten weiterhin einen Zugang zu 2-Oxohexahydropyrimidinen, vgl. DE-A-12 38 921, die als Zwischenprodukte für pharmakologisch wirksame Verbindungen, vgl. J. Am. Chem. Soc. 79, 3786 bis 3788 (1957), verwendet werden können.

Die 1,3-Diurethane der allgemeinen Formel I können weiterhin als Edukt für 1,3-Propandiisocyanate Verwendung finden.

Die nachfolgenden Beispiele dienen zur weiteren Erläuterung der Erfindung.

Beispiel 1

a) 35 g Methacrolein werden innerhalb von 40 min bei 60 bis 65°C zu einem Gemisch aus 133,5 g Ethylcarbamat, 30 g saurem Ionenaustauscherharz (Duolite ®C 265), 0,4 g Hydrochinonmonomethylether und 400 ml Chloroform getropft. Man beläßt das Gemisch 1 Stunde bei dieser Temperatur, filtriert den Ionenaustauscher ab, destilliert Chloroform ab und kristallisiert aus Essigsäureethylester/Petrolether. Man erhält 97 g 2-Methyl-1,1,3-tri(ethoxicarbonylamino)propan (60,8% Ausbeute, bezogen auf eingesetztes Methacrolein) vom Schmp. 138 bis 140°C.

b) 10 g des nach Stufe 1a) erhaltenen 2-Methyl-1,1,3-tri(ethoxicarbonylamino)propans werden in 90 ml Ethanol gelöst, mit 3 g Raney-Nickel versetzt und bei 165°C und 50 bar Wasserstoff während 10 Stunden im Rührautoklaven hydriert. Bei vollständigem Umsatz entsteht 2-Methyl-1,3-di(ethoxicarbonylamino)propan mit einer Selektivität von 95%.

Alternative Arbeitsweise in der zweiten Stufe unter Verwendung von Palladium auf Aktivkohle als Hydrierkatalysator :

10 g des nach Stufe a) erhaltenen 2-Methyl-1,1,3-tri(ethoxicarbonylamino)propans werden in 90 ml Ethanol gelöst, mit 3 g Palladium auf Aktivkohle (10% Pd) versetzt und bei 165°C und 100 bar Wasserstoff 15 Stunden im Rührautoklaven hydriert. Bei vollständigem Umsatz entsteht 2-Methyl-1,3-di(ethoxicarbonylamino)propan mit einer Selektivität von 96%.

Beispiel 2

a) 84 g Ethylacrolein werden innerhalb von 20 min. bei 60 bis 68°C zu einem Gemisch aus 267 g Ethylcarbamat, 30 g saurem Ionenaustauscherharz (Duolite ® C 265), 0,8 g Hydrochinonmonomethylether und 400 ml Chloroform getropft. Nach 60 min wird der Katalysator abfiltriert, das Chloroform abdestilliert und der Rückstand aus Essigsäureethylester/Petrolether kristallisiert. Man erhält 207 g (62,3% Ausbeute, bezogen auf eingesetztes Ethylacrolein) 2-Ethyl-1,1,3-tri(ethoxicarbonylamino)propan vom Schmp. 120 bis 122°C.

b) 130 g des nach Stufe a) hergestellten 2-Ethyl-1,1,3-tri(ethoxicarbonylamino)propans werden in 1300 ml Ethanol gelöst, mit 30 g Raney-Nickel versetzt und 15 Stunden bei 200 bar Wasserstoff und 165°C hydriert. Nach Abfiltrieren des Katalysators und Abdestillieren von Lösungsmittel und Ethylcarbamat erhält man 90 g 2-Ethyl-1,3-di(ethoxicarbonylamino)propan mit einer Reinheit von 94% (88% isolierte Ausbeute).

Beispiel 3

a) 35 g Crotonaldehyd werden innerhalb von 20 min bei 60 bis 83°C zu einem Gemisch aus 133,5 g Ethylcarbamat, 15 g saurem Ionenaustauscherharz (Duolite ® C 265), 0,2 g Hydrochinonmonomethylether und 200 ml Trichlorethylen getropft und anschließend noch 30 min bei 80°C belassen. Nach Abfiltrieren des Katalysators und Abdestillieren des Lösungsmittels wird aus Essigsäureethylester/Petrolether kristallisiert. Man erhält 83 g (52% Ausbeute, bezogen auf eingesetzten Crotonaldehyd) 3-Methyl-1,1,3-tri(ethoxicarbonylamino)propan vom Schmp. 103 bis 105°C.
b) 10 g des nach Beispiel 3a hergestellten 3-Methyl-1,1,3-tri(ethoxicarbonylamino)propans werden mit 3 g Raney-Nickel und 100 ml Ethanol im Rührautoklaven bei 165°C und 200 bar Wasserstoff 15 Stunden hydriert. Bei vollständigem Umsatz entsteht 1-Methyl-1,3-di(ethoxicarbonylamino)propan mit einer Selektivität von 96%.

## Ansprüche

1. Verfahren zur Herstellung von 1,3-Di(alkoxicarbonylamino)propanen der allgemeinen Formel I

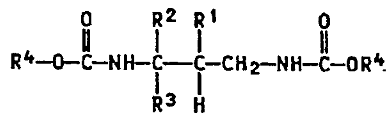

(I),

worin $R^1$, $R^2$ und $R^3$ gleich oder verschieden sein können und jeweils für ein Wasserstoffatom, einen aliphatischen, cycloaliphatischen, araliphatischen oder aromatischen Rest stehen und $R^4$ einen aliphatischen, cycloaliphatischen oder araliphatischen Rest bedeutet, durch Umsetzung eines α,β-ungesättigten Aldehyds mit einem Carbaminsäureester in Gegenwart eines sauren Katalysators, dadurch gekennzeichnet, daß man in einer ersten Stufe als α,β-ungesättigten Aldehyd eine Verbindung der allgemeinen Formel II

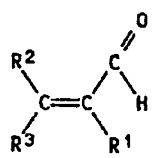

(II),

worin $R^1$, $R^2$ und $R^3$ die vorstehenden Bedeutungen besitzen, mit einem Carbaminsäureester der allgemeinen Formel III

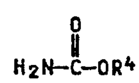

(III),

worin $R^4$ die vorstehende Bedeutung besitzt, bei einer Temperatur von 0 bis 150°C in Gegenwart eines sauren Ionenaustauscherharzes als Katalysator zu einem 1,1,3-Tri(alkoxicarbonylamino)propan der allgemeinen Formel IV

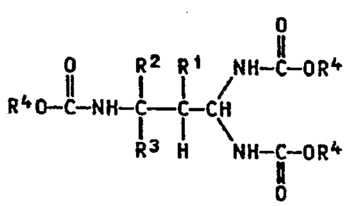

(IV),

worin $R^1$, $R^2$, $R^3$ und $R^4$ die vorstehenden Bedeutungen besitzen, umsetzt,

und in einer zweiten Stufe die Verbindung der allgemeinen Formel (IV) in Gegenwart eines Hydrierkatalysators und Wasserstoff bei einem Druck von 1 bis 300 bar auf eine Temperatur von 100 bis 300°C erhitzt.

2. Verfahren nach Anspruch 1, dadurch gekennzeichnet, daß man in der ersten Stufe bei Temperaturen von 20 bis 120°C und in der zweiten Stufe bei Temperaturen von 130 bis 200°C/10 bis 200 bar, arbeitet.

3. Verfahren nach einem der Ansprüche 1 oder 2, dadurch gekennzeichnet, daß man als Hydrierkatalysator Raney-Nickel oder Palladium auf Aktivkohle einsetzt.

4. Verfahren nach einem der Ansprüche 1 bis 3, dadurch gekennzeichnet, daß man in der ersten Stufe in Gegenwart eines Polymerisationsinhibitors arbeitet.

## Claims

1. A process for preparing a 1,3-di(alkoxycarbonylamino)propane of the general formula I

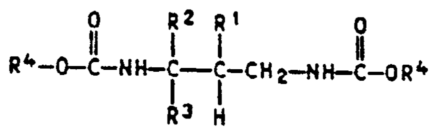

(I)

where $R^1$,$R^2$ and $R^3$ may be identical or different and each is hydrogen, or an aliphatic, cycloaliphatic, araliphatic or aromatic radical, and $R^4$ is an aliphatic, cycloaliphatic or araliphatic radical,

by reacting an α,β-unsaturated aldehyde with a carbamic ester in the presence of an acid catalyst, which comprises reacting in a first stage as the α,β-unsaturated aldehyde a compound of the general formula II

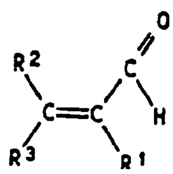

(II)

where $R^1$, $R^2$ and $R^3$ are each as defined above, with a carbamic ester of the general formula III

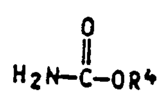

(III)

where $R^4$ is as defined above, in the presence of an acid ion exchanger resin as catalyst, at 0-150°C to give a 1,1,3-tri(alkoxycarbonylamino)propane of the general formula IV

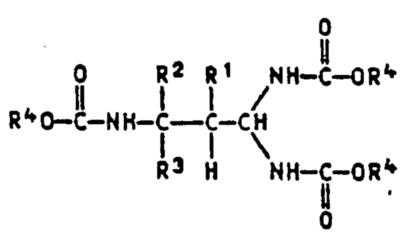

(IV)

where $R^1$, $R^2$, $R^3$ and $R^4$ are each as defined above, and in a second stage heating the compound of the general formula (IV) to 100-300°C in the presence of a hydrogenation catalyst and hydrogen at 1-300 bar.

2. A process as claimed in claim 1, wherein the first stage is carried out at 20-120°C and the second stage at 130-200°C/10-200 bar.

3. A process as claimed in either of claims 1 and 2, wherein the hydrogenation catalyst used is Raney nickel or palladium on activated carbon.

4. A process as claimed in any of claims 1 to 3, wherein the first stage is carried out in the presence of a polymerization inhibitor.

**Revendications**

1. Procédé de préparation de 1,3di(alcoxycarboxylamino)-propanes de formule générale I

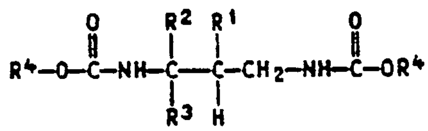

(I),

dans laquelle $R^1$, $R^2$ et $R^3$ peuvent être identiques ou différents et sont chaque fois mis pour un atome d'hydrogène ou un reste aliphatique, cycloaliphatique, araliphatique ou aromatique et $R^4$ représente un reste aliphatique, cycloaliphatique ou araliphatique, par réaction d'un aldéhyde α,β-insaturé avec un ester d'acide carbamique en présence d'un catalyseur acide, caractérisé en ce qu'on fait réagir, dans une première étape, comme aldéhyde α,β-insaturé, un composé de formule générale II

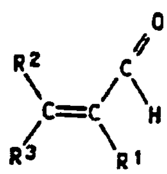

(II),

dans laquelle $R^1$, $R^2$ et $R^3$ sont tels que définis précédemment, avec un ester d'acide carbamique de formule générale III

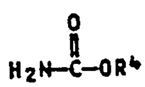

(III),

dans laquelle $R^4$ est tel que défini précédemment, à une température de 0 à 150°C en présence d'une résine échangeuse d'ions acide comme catalyseur, pour former un 1,1,3-(trialcoxy-carboxylamino)propane de formule générale IV

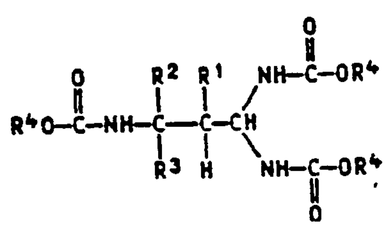

(IV),

dans laquelle $R^1$, $R^2$, $R^3$ et $R^4$ sont tels que définis précédemment,

et dans une deuxième étape, on chauffe le composé de formule générale IV en présence d'un catalyseur d'hydrogénation et d'hydrogène sous une pression de 1 à 300 bar et une température de 100 à 300°C.

2. Procédé selon la revendication 1, caractérisé en ce qu'on travaille dans la première étape à des températures de 20 à 120°C et dans la deuxième étape à des températures de 130 à 200°C/10 à 200 bar.

3. Procédé selon l'une ou l'autre des revendications 1 ou 2, caractérisé en ce qu'on utilise comme catalyseur d'hydrogénation du nickel de Raney ou du palladium sur charbon actif.

4. Procédé selon l'une quelconque des revendications 1 à 3, caractérisé en ce qu'on travaille dans la première étape en présence d'un inhibiteur de polymérisation.